# EUROPEAN PATENT SPECIFICATION

(11) **EP 1 147 775 B1**
(45) Date of publication and mention of the grant of the patent: **04.03.2009**
(21) Application number: 01118792.9
(22) Date of filing: 16.12.1994
(51) Int. Cl.: A61K 38/16, A61P 21/02

(54) **Use of botulinum toxin type B for the manufacture of a medicament for reducing pain associated with a muscle disorder**
Verwendung von Botulinumtoxin Typ B zur Herstellung eines Medikaments zur Reduzierung von Schmerzen bedingt durch Muskelkrankheit
L'utilisation de la toxine botulinique type B pour la préparation d'un médicament à réduire la douleur liée à des troubles musculaires

(30) Priority: 28.12.1993 US 173996
(43) Date of publication of application: 24.10.2001
(62) Divisional of application: 00203294.4
(73) Proprietor: ALLERGAN, INC., Irvine CA 92612 (US)
(72) Inventor: Aoki, K. Roger, Laguna Hills, CA 92653 (US); Grayston, Michael W., Irvine, CA 92714 (US); Carlson, Steven R., Laguna Niguel, CA 92677 (US); Leon, Judith M., Laguna Niguel, CA 92677 (US)
(74) Representative: HOFFMANN EITLE

(56) References cited:
- WO-A-94/00481
- WO-A-94/28922
- US-A- 5 696 077
- BORODIC G E ET AL: "Botulinum B toxin as an alternative to botulinum A toxin: a histologic study." OPHTHALMIC PLASTIC AND RECONSTRUCTIVE SURGERY. UNITED STATES 1993, vol. 9, no. 3, 1993, pages 182-190, XP009000956 ISSN: 0740-9303
- SELLIN L C ET AL: "DIFFERENT EFFECTS OF TYPE A AND B BOTULINUM TOXIN ON TRANSMITTER RELEASE AT THE RAT NEURO MUSCULAR JUNCTION" ACTA PHYSIOLOGICA SCANDINAVICA, vol. 119, no. 2, 1983, pages 127-134, XP009000970 ISSN: 0001-6772
- SCHANTZ E J ET AL: "PROPERTIES AND USE OF BOTULINUM TOXIN AND OTHER MICROBIAL NEUROTOXINS IN MEDICINE" MICROBIOLOGICAL REVIEWS, AMERICAN SOCIETY FOR MICROBIOLOGY, WASHINGTON, DC, US, vol. 56, no. 1, 1 March 1992 (1992-03-01), pages 80-99, XP000569909 ISSN: 0146-0749
- ANDERSON TJ ET AL.: "Botulinum toxin treatment of spasmodic torticollis" THE ROYAL SOCIETY OF MEDICINE, vol. 85, September 1992 (1992-09), pages 524-529, XP009019478
- DATABASE STN Genesis Report -RX, Feb 1993, Vol 2, No. 2 February 1993 (1993-02), "Focal dystonia : In Phase I/II clinicals" XP002199878

## Description

The present invention relates to Botulinum toxin type B. Importantly, the present invention relates to relieving pain related to muscle activity or contracture and therefore is of advantage in the treatment of, for example, muscle spasm such as Temporomandibular Joint Disease, low back pain, myofascial pain, pain related to spasticity and dystonia, as well as sports injuries, and pain related to contractures in arthritis.

Heretofore, Botulinum toxins, in particular Botulinum toxin type A, has been used in the treatment of a number of neuromuscular disorders and conditions involving muscular spasm; for example, strabismus, blepharospasm, spasmodic torticollis (cervical dystonia), oromandibular dystonia and spasmodic dysphonia (laryngeal dystonia). The toxin binds rabidly and strongly to presynaptic cholinergic nerve terminal and inhibits the exocytosis of acetylcholine by decreasing the frequency of acetylcholine release. This results in local paralysis and hence relaxation of the muscle afflicted by spasm.

For one example of treating neuromuscular disorders, see U.S. Patent No. 5,053,005 to Borodic, which suggests treating' curvature of the juvenile spine, i.e., scoliosis, with an acetylcholine release inhibitor, preferably Botulinum toxin A.

For the treatment of strabismus with Botulinum toxin type A, see Elston, J.S., et al., British Journal of Ophthalmology, 1985, 69, 718-724 and 891-896. For the treatment of blepharospasm with Botulinum toxin type A, see Adenis, J.P., et al., J. Fr. Ophthalmol., 1990, 13 (5) at pages 259-264. For treating squint, see Elston, J.S., Eye, 1990, 4(4):VII. For treating spasmodic and oromandibular dystonia torticollis, see Jankovic et al., Neurology, 1987, 37, 616-623.

Spasmodic dysphonia has been treated with Botulinum toxin type A. See Blitzer et al., Ann. Otol. Rhino. Laryngol, 1985, 94, 591-594. Lingual dystonia was treated with Botulinum toxin type A according to Brin et al., Adv. Neurol. (1987) 50, 599-608. Finally, Cohen et al., Neurology (1987) 37 (-Suppl. 1), 123-4, discloses the treatment of writer's cramp with Botulinum toxin type A.

The term Botulinum toxin is a generic term embracing the family of toxins produced by the anaerobic bacterium *Clostridium botulinum* and, to date, seven immunologically distinct neurotoxins have been identified. These have been given the designations A, B, C, D, E, F and G. For further information concerning the properties of the various Botulinum toxins, reference is made to the article by Jankovic and Brin, The New England Journal of Medicine, No. 17, 1990, pp. 1186-1194, and to the review by Charles L. Hatheway in Chapter 1 of the book entitled Botulinum Neurotoxin and Tetanus Toxin, L. L. Simpson, Ed., published by Academic Press Inc. of San Diego, California, 1989, the disclosures in which are incorporated herein by reference.

The neurotoxic component of Botulinum toxin has a molecular weight of about 150 kilodaltons and is thought to comprise a short polypeptide chain of about 50 kD which is considered to be responsible for the toxic properties of the toxin, i.e., by interfering with the exocytosis of acetylcholine, by decreasing the frequency of acetylcholine release, and a larger polypeptide chain of about 100 kD which is believed to be necessary to enable the toxin to bind to the presynaptic membrane.

The "short" and "long" chains are linked together by means of a simple disulfide bridge. (It is noted that certain serotypes of Botulinum toxin, e.g., type E, may exist in the form of a single chain un-nicked protein, as opposed to a dichain. The single chain form is less active but may be converted to the corresponding dichain by nicking with a protease, e.g., trypsin. Both the single and the dichain are useful in the method of the present invention.)

In general, four physiologic groups of *C*. *botulinum* are recognized (I, II, III, IV). The organisms capable of producing a serologically distinct toxin may come from more than one physiological group. For example, Type B and F toxins can be produced by strains from Group I or II. In addition, other strains of clostridial species *(C. baratii,* type F; *C. butyricum,* type E; *C. novyi,* type C₁ or D) have been identified which can produce botulinum neurotoxins.

Immunotoxin conjugates of ricin and antibodies, which are characterized as having enhanced cytotoxicity through improving cell surface affinity, are disclosed in European Patent Specification 0 129 434. The inventors note that botulinum toxin may be utilized in place of ricin.

Botulinum toxin is obtained commercially by establishing and growing cultures of *C*. *botulinum* in a fermenter and then harvesting and purifying the fermented mixture in accordance with known techniques.

Botulinum toxin type A, the toxin type generally utilized in treating neuromuscular conditions, is currently available commercially from several sources; for example, from Porton Products Ltd. UK, under the trade name "DYSPORT," and from Allergan, Inc., Irvine, California, under the trade name BOTOX^{®}**.**

It is one object of the invention to provide novel treatments of neuromuscular disorders and conditions with various Botulinum toxin types. It is another object of the present invention to relieve pain with various Botulinum toxin types.

### SUMMARY OF THE INVENTION

The present invention relates to the use of Botulinum toxin type B in a medicament for relieving pain, associated with muscle contractions, and smooth muscle disorders including, but not limited to, spasms in the sphincter of the cardiovascular arteriole, gastrointestinal system, urinary, gall bladder and rectum.

Each serotype of Botulinum toxin has been identified as immunologically different proteins through the use of specific antibodies. For example, if the antibody (antitoxin) recognizes, that is, neutralizes the biological activity of, for example, type A it will not recognize types B,C,D, E, F or G.

While all of the Botulinum toxins appear to be zinc endopeptidases, the mechanism of action of different serotypes, for example, A and E within the neuron appear to be different than that of Type B. In addition, the neuronal surface "receptor" for the toxin appears to be different for the serotypes.

In the area of use of the Botulinum toxin in accordance with the present invention with regard to organ systems which involve the release of neurotransmitter, it is expected to introduce the toxin type B directly by loyal injections.

The Botulinum toxin used according to the present invention is Botulinum toxins type B.

The physiologic groups of *Clostridium botulinum* types are listed in Table I.

**Table I. Physiologic Groups of Clostridium botulinum**

| Group | Toxin Sero-Type | Biochemistry | Milk Digest | Glucose Permentation | Lipase | Phages & Plasmis | Phenotypically Related Clostridium (nontoxigenie) |
|---|---|---|---|---|---|---|---|
| I | A,B,F | proteolysic saccharolytic | + | + | + | + | C sporogenes |
| II | B,E,F | nonproteolytic saccharolytic psychotraphic | - | + | + | + | |
| III | C,D | nonproteolytic saccharolytic | + | + | + | + | C. novvi |
| IV | G | protecolytic nonsaccharolytic | + | - | - | - | C subterminate |

These toxin types may be produced by selection from the appropriate physiologic group of *Clostridium botulinum* organisms. the organisms designated as Group I are usually referred to as proteolytic and produce Botulinum toxins of types A, B and F. The organisms designated as Group II are saccharolytic and produce Botulinum toxins of types B, E and F. The organisms designated as Group III produce only Botulinum toxin types C and D and are distinguished from organisms of Groups I and II by the production of significant amounts of propionic acid. Group IV organisms only produce neurotoxin of type G. The production of any and all of the Botulinum toxin types A, B, C, D, E, F and G are described in Chapter 1 of *Botulinum Neurotoxin and Tetanus Toxin,* cited above, and/or the references cited therein. Botulinum toxins types B, C, D, E, F and G are also available from various species of clostridia.

Currently fourteen species of clostridia are considered pathogenic. Most of the pathogenic strains produce toxins which are responsible for the various pathological signs and symptoms. Organisms which produce Botulinum toxins have been isolated from botulism outbreaks in humans (types A, B, E and F) and animals (types C and D). Their identities were described through the use of specific antitoxins (antibodies) developed against the earlier toxins. Type G toxin was found in soil and has low toxigenicity. However, it has been isolated from autopsy specimens, but thus far there has not been adequate evidence that type G botulism has occurred in humans.

Preferably, the toxin is administered by means of intramuscular injection directly into a local area such as a spastic muscle, preferably in the region of the neuromuscular junction, although alternative types of administration (e.g., subcutaneous injection), which can deliver the toxin directly to the affected region, may be employed where appropriate. The toxin can be presented as a sterile pyrogen-free aqueous solution or dispersion and as a sterile powder for reconstitution into a sterile solution or dispersion.

Where desired, tonicity adjusting agents such as sodium chloride, glycerol and various sugars can be added. Stabilizers such as human serum albumin may also be included. The formulation may be preserved by means of a suitable pharmaceutically acceptable preservative such as a paraben, although preferably it is unpreserved.

It is preferred that the toxin is formulated in unit dosage form; for example, it can be provided as a sterile solution in a vial or as a vial or sachet containing a lyophilized powder for reconstituting a suitable vehicle such as saline for injection.

In one embodiment, the Botulinum toxin is formulated in a solution containing saline and pasteurized human serum albumin, which stabilizes the toxin and minimizes loss through non-specific adsorption. The solution is sterile filtered (0.2 micron filter), filled into individual vials and then vacuum-dried to give a sterile lyophilized powder. In use, the powder can be reconstituted by the addition of sterile unpreserved normal saline (sodium chloride 0.9% for injection).

The dose of toxin administered to the patient will depend upon the severity of the condition; e.g., the number of muscle groups requiring treatment, the age and size of the patient and the potency of the toxin. The potency of the toxin is expressed as a multiple of the IM₅₀ value for the mouse, one unit (U) of toxin being defined as being the equivalent amount of toxin that kills 50% of a group of 18 to 20 female Swiss-Webster mice, weighing about 20 grams each.

The dosages used in human therapeutic applications are roughly proportional to the mass of muscle being injected. Typically, the dose administered to the patient may be up from about 0.01 to about 1,000 units; for example, up to about 500 units, and preferably in the range from about 80 to about 460 units per patient per treatment, although smaller of larger doses may be administered in appropriate circumstances such as up to about 50 units for the relief of pain and in controlling cholinergic secretions.

As the physicians become more familiar with the use of this product, the dose may be changed. The potency of Botulinum toxin and its long duration of action mean that doses will tend to be administered on an infrequent basis. Ultimately, however, both the quantity of toxin administered and the frequency of its administration will be at the discretion of the physician responsible for the treatment and will be commensurate with questions of safety and the effects produced by the toxin.

The invention will now be illustrated by reference to the following nonlimiting examples.

In each of the examples, appropriate areas of each patient are injected with a sterile solution containing the confirmation of Botulinum toxin. Total patient doses range from about 0.01 units to 460 units. Before injecting any muscle group, careful consideration is given to the anatomy of the muscle group, the aim being to inject the area with the highest concentration of neuromuscular junctions, if known. Before injecting the muscle, the position of the needle in the muscle is confirmed by putting the muscle through its range of motion and observing the resultant motion of the needle end. General anaesthesia, local anaesthesia and sedation are used according to the age of the patient, the number of sites to be injected, and the particular needs of the patient. More than one injection and/or sites of injection may be necessary to achieve the desired result. Also, some injections, depending on the muscle to be injected, may require the use of fine, hollow, teflon-coated needles, guided by electromyography.

Following injection, it is noted that there are no systemic or local side effects and none of the patients are found to develop extensive local hypotonicity. The majority of patients show an improvement in function both subjectively and when measured objectively.

### Example 1

### The Use of Botulinum toxin in the Treatment of Muscle spasms in Smooth Muscle Disorders Such As Sphincters of the cardiovascular Arteriole, Gastrointestinal System, Urinary or Gall Bladder, Rectal, Etc.

A male, age 30-40, with a constricted pyloric valve which prevents his stomach from emptying, is treated by administering 1-50 units of Botulinum toxin. The administration is to the pyloric valve (which controls release of stomach contents into the intestine) divided into 2 to 4 quadrants, injections made with any endoscopic device or during surgery. In about 1-7 days, normal emptying of the stomach, elimination or drastic reduction in regurgitation occurs.

### Example 2

### The Use of Botulinum toxin in the Treatment of Muscle Spasms and control of Pain Associated with Muscle Spasms in Temporal Mandibular Joint Disorders

A female, age 35, is treated by administration of 0.1 to 50 units total of Botulinum toxin. The administration is to the muscles controlling the closure of the jaw. Overactive muscles may be identified with EMG (electromyography) guidance. Relief of pain associated with muscle spasms, possible reduction in jaw clenching occurs in about 1-3 days.

### Example 3

### The Use of Botulinum toxin in the Treatment of Muscle Spasms and Control of Pain Associated with Muscle Spasms in Smooth Muscle Disorders Such as Gastrointestinal Muscles

A female, age 35, with spastic colitis, is treated with 1-100 units of Botulinum toxin divided into several areas, enema (1-5 units) delivered in the standard enema volume, titrate dose, starting with the lowest dose. Injection is to the rectum or lower colon or a low dose enema may be employed. Cramps and pain associated with spastic colon are relieved in 1-10 days.

### Example 4

### The Use of Botulinum toxin in the Treatment of Muscle Spasms and Control of Pain Associated with Muscle Spasms in Spasticity Conditions secondary to stroke, Traumatic Brain or Spinal Cord Injury

A male, age 70, post-stroke or cerebral vascular event, is injected with 50 to 300 units of Botulinum toxin in the major muscles involved in severe closing of hand and curling of wrist and forearm or the muscles involved in the closing of the legs such that the patient and attendant have difficulty with hygiene. Relief of these symptoms occurs in 7 to 21 days.

## Claims

1. Use of botulinum toxin type B for the manufacture of a medicament for reducing pain associated with a muscle activity or contraction.

2. Use according to claim 1, wherein the muscle activity or contraction is a muscle spasm or a spasticity condition.

3. Use according to claim 2, wherein the spasticity condition is secondary to a stroke or a cerebral vascular event.

4. Use according to claim 2, wherein the spasticity condition is secondary to a traumatic brain injury.

5. Use according to claim 2, wherein the spasticity condition is secondary to a spinal cord injury.

6. Use according to claim 2, wherein the muscle spasm is associated with Temporomandibular Joint Disorder.

7. Use according to claim 1, wherein the pain is associated with a smooth muscle disorder.

8. Use according to claim 7, wherein the smooth muscle disorder is a constricted pyloric valve.

9. Use according to claim 7, wherein the smooth muscle disorder is spastic colitis.

10. Use according to claim 7, wherein the smooth muscle disorder is a spasmodic muscle.

11. Use according to claim 10, wherein the spasmodic muscle is in the gastrointestinal system.

12. Use according to claim 10, wherein the spasmodic muscle is in the colon.

13. Use according to claim 10, wherein the spasmodic muscle is a sphincter of a cardiovascular arteriole.

14. Use according to claim 10, wherein the spasmodic muscle is a sphincter in the gastrointestinal system.

15. Use according to claim 10, wherein the spasmodic muscle is a sphincter in the urinary system.

16. Use according to claim 10, wherein the spasmodic muscle is a sphincter of the gall bladder.

17. Use according to claim 10, wherein the spasmodic muscle is a rectal sphincter.

18. Use according to claim 2, wherein the pain is associated with a muscle spasm in a condition secondary to a sports injury.

19. Use according to claim 18, wherein the muscle spasm occurs in the patient's thigh and the botulinum toxin is formulated for admininstration into the thigh.

20. Use according to claim 1, wherein the pain is related to contractures in arthritis.

21. Use according to claim 1, wherein the pain is lower back pain.

22. Use according to claim1, wherein the pain is myofascial pain

23. Use according to claim 1, wherein the pain is related to spasticity.

24. Use according to claim 1, wherein the botulinum toxin is formulated for administration in an amount of at least about 1,000 units.

25. Use according to claim 1, wherein the botulinum toxin is formulated for intramuscular injection.

26. Use according to claim 1, wherein the muscle activity or contraction is a cholinergic influenced muscle activity or contraction.

## Patentansprüche

1. Verwendung von Botulinumtoxin vom Typ B zur Herstellung eines Medikaments zur Verringerung von Schmerzen, die mit einer Muskelaktivität oder -kontraktion verbunden sind.

2. Verwendung gemäß Anspruch 1, wobei die Muskelaktivität oder -kontraktion ein Muskelspasmus oder ein spastischer Zustand ist.

3. Verwendung gemäß Anspruch 2, wobei der spastische Zustand sekundär zu einem Schlaganfall oder einem cerebro-vaskulären Vorfall ist.

4. Verwendung gemäß Anspruch 2, wobei der spastische Zustand sekundär zu einer traumatischen Gehirnverletzung ist.

5. Verwendung gemäß Anspruch 2, wobei der spastische Zustand sekundär zu einer Rückenmarksverletzung ist.

6. Verwendung gemäß Anspruch 2, wobei der Muskelspasmus mit einer kraniomandibulären Dysfunktion verbunden ist.

7. Verwendung gemäß Anspruch 1, wobei der Schmerz mit einer Erkrankung des Glattmuskelgewebes verbunden ist.

8. Verwendung gemäß Anspruch 7, wobei die Erkrankung des Glattmuskelgewebes ein verengter Pylor ist.

9. Verwendung gemäß Anspruch 7, wobei die Erkrankung des Glattmuskelgewebes eine spastische Kolitis ist.

10. Verwendung gemäß Anspruch 7, wobei die Erkrankung des Glattmuskelgewebes ein spasmodischer Muskel ist.

11. Verwendung gemäß Anspruch 10, wobei sich der spasmodische Muskel im gastrointestinalen System befindet.

12. Verwendung gemäß Anspruch 10, wobei der spasmodische Muskel im Dickdarm ist.

13. Verwendung gemäß Anspruch 10, wobei der spasmodische Muskel ein Schließmuskel einer kardiovaskulären Arteriole ist.

14. Verwendung gemäß Anspruch 10, wobei der spasmodische Muskel ein Schließmuskel im gastrointestinalen System ist.

15. Verwendung gemäß Anspruch 10, wobei der spasmodische Muskel ein Schließmuskel in Harnorganen ist.

16. Verwendung gemäß Anspruch 10, wobei der spasmodische Muskel ein Schließmuskel der Gallenblase ist.

17. Verwendung gemäß Anspruch 10, wobei der spasmodische Muskel ein rektaler Schließmuskel ist.

18. Verwendung gemäß Anspruch 2, wobei der Schmerz mit einem Muskelspasmus in einem Zustand, der sekundär zu einer Sportverletzung ist, verbunden ist.

19. Verwendung gemäß Anspruch 18, wobei der Muskelspasmus im Oberschenkel des Patienten auftritt und das Botulinumtoxin für die Verabreichung in den Oberschenkel formuliert ist.

20. Verwendung gemäß Anspruch 1, wobei der Schmerz mit Kontraktionen bei Arthritis verbunden ist.

21. Verwendung gemäß Anspruch 1, wobei der Schmerz ein Schmerz im unteren Rückenbereich ist.

22. Verwendung gemäß Anspruch 1, wobei der Schmerz ein myofaszialer Schmerz ist.

23. Verwendung gemäß Anspruch 1, wobei der Schmerz mit der Spastik zusammenhängt.

24. Verwendung gemäß Anspruch 1, wobei das Botulinumtoxin für die Verabreichung in einer Menge von mindestens circa 1.000 Einheiten formuliert ist.

25. Verwendung gemäß Anspruch 1, wobei das Botulinumtoxin für eine intramuskuläre Injektion formuliert ist.

26. Verwendung gemäß Anspruch 1, wobei die Muskelaktivität oder -kontraktion eine cholinergisch beeinflußte Muskelaktivität oder -kontraktion ist.

## Revendications

1. Utilisation de la toxine botulinique de type B pour la fabrication d'un médicament destiné à réduire la douleur associée à une activité ou à une contraction musculaire.

2. Utilisation selon la revendication 1, dans laquelle l'activité ou la contraction musculaire est un spasme musculaire ou un trouble de spasticité.

3. Utilisation selon la revendication 2, dans laquelle le trouble de spasticité est consécutif à un accident cérébrovasculaire ou à un évènement cérébrovasculaire.

4. Utilisation selon la revendication 2, dans laquelle le trouble de spasticité est consécutif à une lésion traumatique du cerveau.

5. Utilisation selon la revendication 2, dans laquelle le trouble de spasticité est consécutif à une blessure médullaire.

6. Utilisation selon la revendication 2, dans laquelle le spasme musculaire est associé au syndrome de l'articulation temporo-mandibulaire.

7. Utilisation selon la revendication 1, dans laquelle la douleur est associée à un trouble du muscle lisse.

8. Utilisation selon la revendication 7, dans laquelle le trouble du muscle lisse est une valve pylorique comprimée.

9. Utilisation selon la revendication 7, dans laquelle le trouble du muscle lisse est une colite spastique.

10. Utilisation selon la revendication 7, dans laquelle le trouble du muscle lisse est un muscle spasmodique.

11. Utilisation selon la revendication 10, dans laquelle le muscle spasmodique est dans le système gastro-intestinal.

12. Utilisation selon la revendication 10, dans laquelle le muscle spasmodique est dans le côlon.

13. Utilisation selon la revendication 10, dans laquelle le muscle spasmodique est un sphincter d'une artériole cardiovasculaire.

14. Utilisation selon la revendication 10, dans laquelle le muscle spasmodique est un sphincter dans le système gastro-intestinal.

15. Utilisation selon la revendication 10, dans laquelle le muscle spasmodique est un sphincter dans le système urinaire.

16. Utilisation selon la revendication 10, dans laquelle le muscle spasmodique est un sphincter de la vésicule biliaire.

17. Utilisation selon la revendication 10, dans laquelle le muscle spasmodique est un sphincter rectal.

18. Utilisation selon la revendication 2, dans laquelle la douleur est associée à un spasme musculaire lié à un trouble consécutif à une blessure sportive.

19. Utilisation selon la revendication 18, dans laquelle le spasme musculaire a lieu dans la cuisse du patient et la toxine botulinique est formulée pour une administration dans la cuisse.

20. Utilisation selon la revendication 1, dans laquelle la douleur est associée à des contractures liées à l'arthrite.

21. Utilisation selon la revendication 1, dans laquelle la douleur est une douleur de la partie inférieure du dos.

22. Utilisation selon la revendication 1, dans laquelle la douleur est une douleur myofasciale.

23. Utilisation selon la revendication 1, dans laquelle la douleur est associée à la spasticité.

24. Utilisation selon la revendication 1, dans laquelle la toxine botulinique est formulée pour une administration en une quantité supérieure ou égale à environ 1 000 unités.

25. Utilisation selon la revendication 1, dans laquelle la toxine botulinique est formulée pour une injection intramusculaire.

26. Utilisation selon la revendication 1, dans laquelle l'activité ou la contraction musculaire est une activité ou une contraction musculaire d'influence cholinergique.
